# EUROPEAN PATENT SPECIFICATION

(11) **EP 1 778 660 B1**
(45) Date of publication and mention of the grant of the patent: **28.01.2009**
(21) Application number: 05770208.6
(22) Date of filing: 01.07.2005
(51) Int. Cl.: C07D 305/14

(54) **PROCESS FOR THE SEPARATION OF PACLITAXEL AND CEPHALOMANNIN**
VERFAHREN ZUR TRENNUNG VON PACLITAXEL UND CEPHALOMANNIN
PROCESSUS DE SÉPARATION DE PACLITAXEL ET DE CÉPHALOMANNINE

(30) Priority: 02.07.2004 US 585402 P
(43) Date of publication of application: 02.05.2007
(73) Proprietor: IVAX Pharmaceuticals s.r.o., 747 70 Opava 9 (CZ)
(72) Inventor: BUCHTA, Martin, Ludgerovice 747 14 (CZ); CVAK, Ladislav, 746 01 Opava - Zlatniky (CZ); SOBOTIK, Roman, Opava 7, 747 07 (CZ); STVERKA, Pavel, Velka Polom 747 64 (CZ)
(74) Representative: Gallagher, Kirk James
(86) International application number: PCT/US2005/023483
(87) International publication number: WO 2006/014345

(56) References cited:
- WO-A-02/38556
- US-B1- 6 333 419

## Description

### FIELD OF THE INVENTION

The present invention relates to a process for the separation of paclitaxel from its natural analogue, cephalomannin. In particular, the present invention relates to the separation of paclitaxel from cephalomannin by silica gel chromatography.

### BACKGROUND

Paclitaxel, formerly known as "taxol", is an important chemotherapeutic agent useful for the treatment of human ovarian, breast and lung tumors. It has shown promise for a number of human cancers and its clinical uses have been reported in several review articles, such as Rowinsky, E. K., Ann. Rev. Med. 48:353 1997; Van Hoff, D. D., Semin. Oncol. 24:3 (1997); DeFuria, M. D., Phytomedicine 4:273 (1997); and Eisenhauer, E. A., Vermorken, J. B., Drugs 55:5 (1998).

Paclitaxel is a natural compound and was first isolated by Wani, et al. from the bark of Pacific yew (Taxus brevifolia). J. Am. Chem. Soc. 93:2325 (1971). Since that time, researchers have recognized that paclitaxel exists in all other species of the *Taxus* genus, including European yew *(Taxus baccata),* Himalayan yew (*Taxus Wallichiana*)*,* Chinese yew *(Taxus celebita),* Japanese yew *(Taxus cuspidata),* Canadian yew *(Taxus canadensis),* Mexican yew (*Taxus globosa*), Florida yew *(Taxus floridana)* and ornamental yew *(Taxus media)* and all of their hybrids and cultivars.

The isolation of paclitaxel from vegetative sources is complex and difficult, partly due to its very low biomass concentration and partly due to the presence of other taxanes having a similar structure and properties related to paclitaxel. The taxane, cephalomannin is particularly difficult to separate from paclitaxel. It is present in virtually all known sources containing paclitaxel and ranges in concentration from a paclitaxel/ cephalomannin ratio between about 1:10 to as high as about 1:1, depending on the type of *Taxus* plant.

While other taxane impurities can be separated from paclitaxel by crystallization, cephalomannin tends to crystallize with paclitaxel. Cephalomannin and paclitaxel differ in structure by only one side chain group. Their structures are reproduced below for comparison.

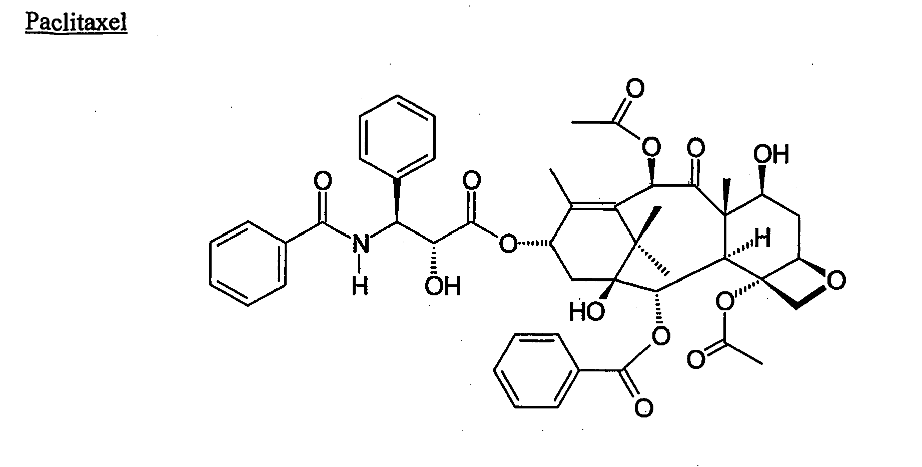

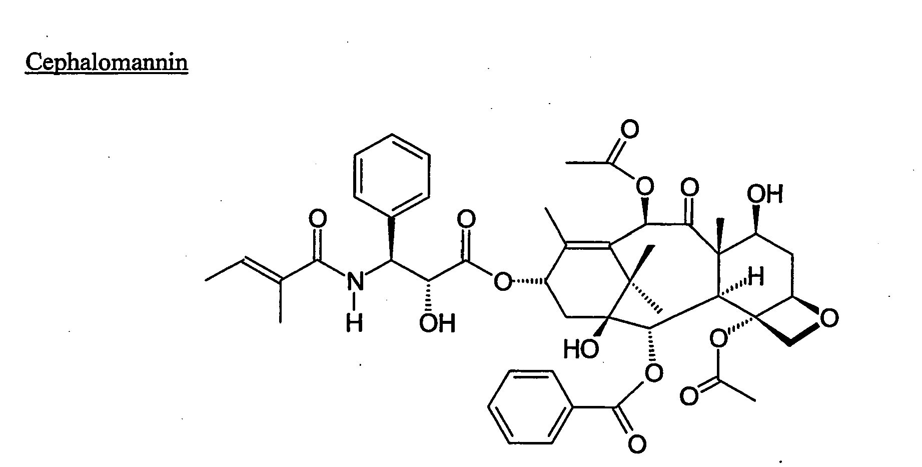

One method used to separate these compounds exploits the difference in their chemical reactivity to form derivatives of the more reactive cephalomannin. For example, the olefinic group extending from cephalomannin is more readily oxidized or brominated than any part of paclitaxel and can be used to form a cephalomannin derivative that is easier to separate from paclitaxel. See, *e.g.*, Kingston, D. G. I., et al., J. Nat. Prod. 55:259 (1992)); Beckvermit, J. T., et al., J. Org. Chem. 61:9038 (1996); U.S. Patent No. 5,654,448.

Another approach to the separation of the cephalomannin from paclitaxel involves various types of chromatography. Numerous examples can be found in the literature, including, for example, Dauh-Rurng Wu, et al., J. Chrom. A, 702:233 (1995); Koppaka V. Rao, et al., Pharm. Res. 12:1003 (1995); and Xuefeng Yang ,et al., J. Chrom. A, 813:201 (1998). Reverse phase chromatography is also described for the final purification of paclitaxel in several patents, such as: U.S. Patent No. 5,279,949; U.S. Patent No. 5,380,916; and U.S. Patent No. 5,969,165. A continuous process was also developed based on a simulated moving-bed reverse phase chromatography procedure. Dauh-Rurng Wu, et al., J. Chrom. A, 855:71 (1999). However, a disadvantage of using reverse phase chromatography is the need for water containing solvents, which can adversely cause the isomerization of paclitaxel to undesired 7-epi-paclitaxel.

Additional methods being used to purify paclitaxel are based either on complicated gradient elution or several sequential chromatographic steps using different mobile phase compositions. Young Kwang Park, et al., J. Liq. Chrom. & Rel. Technol. 22(18):2755 (1999). Another approach uses normal phase chromatography with alumina as the adsorbent. Unfortunately, chlorinated organic solvents are often used as the mobile phase with alumina. Moreover, both epimerization and chemical decomposition of paclitaxel take place in this system, depending on the elution time. Zhiqiang Z., Zhiguo S. J., Liq. Chrom. & Rel. Technol. 23(17):2683 (2000).

Recently, the chromatographic purification of paclitaxel on silica using organic esters was described in U.S. Patent No. 6,333,419. The process described therein was designed for the separation of paclitaxel from cephalomannin.

There is still a continuing need for an effective and simple method for separating paclitaxel and cephalomannin.

### SUMMARY OF THE DISCLOSURE

An advantage of the present invention is a simple, inexpensive and effective method for separating paclitaxel from cephalomannin.

These and other advantages are satisfied, at least in part, by a process of separating paclitaxel from cephalomannin employing silica gel chromatography. The process can be advantageously adapted for large-scale purification of paclitaxel.

Embodiments of the present invention include applying a cephalomannin and paclitaxel composition to a container, *e.g.*, a column, comprising silica and then applying a solvent mixture comprising methyl isobutyl ketone together with a less polar solvent to the container. The solvent mixture is added to the container to cause it and at least paclitaxel and cephalomannin to separately elute from the container. One or more fractions of the eluting solvent mixture containing paclitaxel are then collected. If desired, fractions containing cephalomannin can also be separately collected. Pure paclitaxel can then be isolated from the appropriate fractions containing the highest concentrations of paclitaxel by evaporation of the solvent mixture and crystallization of the residue, if desired.

Additional advantages of the present invention will become readily apparent to those skilled in this art from the following detailed description, wherein only the preferred embodiment of the invention is shown and described, simply by way of illustration of carrying out the invention. As will be realized, the invention is capable of other and different embodiments, and its several details are capable of modifications in various obvious respects, all without departing from the invention. The present invention may be practiced without some or all of these specific details. In other instances, well known process operations have not been described in detail, in order not to unnecessarily obscure the present invention. Accordingly, the drawings and description are to be regarded as illustrative in nature, and not as restrictive.

### BRIEF DESCRIPTION OF THE DRAWINGS

Reference is made to the attached drawings, wherein:

Fig. 1 illustrates the composition of the starting material used in Examples 1, 3, and 4, as determined by HPLC analysis, wherein the abscissa represents the elution time in minutes, and the ordinate represents absorbance units.

Fig. 2 illustrates the composition of the main fraction of Example 1, as determined by HPLC analysis, wherein the abscissa represents the elution time in minutes, and the ordinate represents absorbance units.

Fig. 3 illustrates the composition of the final crystalline product of Example 1, as detemined by HPLC analysis, wherein the abscissa represents the elution time in minutes, and the ordinate represents absorbance units.

Fig. 4 shows the composition of the starting material used in Example 2, as determined by HPLC analysis, wherein the abscissa represents the elution time in minutes, and the ordinate represents absorbance units.

Fig. 5 illustrates the composition of the final crystalline product of Example 2, as detemined by HPLC analysis, wherein the abscissa represents the elution time in minutes, and the ordinate represents absorbance units.

Fig. 6 illustrates the composition of the main fractions of Example 3, as detemined by HPLC analysis, wherein the abscissa represents the elution time in minutes, and the ordinate represents absorbance units.

Fig. 7 illustrates the composition of the main fractions of Example 4, as detemined by HPLC analysis, wherein the abscissa represents the elution time in minutes, and the ordinate represents absorbance units.

### DETAILED DESCRIPTION OF THE DISCLOSURE

The present invention stems from the discovery that paclitaxel can be effectively separated from cephalomannin by silica gel chromatography using a solvent mixture comprising methyl isobutyl ketone and another less polar solvent as the mobile phase. The process provides a simple, inexpensive and effective method for the production of high purity paclitaxel, *e.g.*, a paclitaxel product having less than about 0.5% of cephalomannin, which can be adapted for industrial scale manufacture.

The patents, published applications, and scientific literature referred to herein establish the knowledge of those with skill in the art and are hereby incorporated by reference in their entirety to the same extent as if each was specifically and individually indicated to be incorporated by reference. Any conflict between any reference cited herein and the specific teachings of this specification shall be resolved in favor of the latter. Likewise, any conflict between an art-understood definition of a word or phrase and a definition of the word or phrase as specifically taught in this specification shall be resolved in favor of the latter.

As used in this specification, the singular forms "a," "an" and "the" specifically also encompass the plural forms of the terms to which they refer, unless the context clearly dictates otherwise.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, the recitation of a numerical range for a variable is intended to convey that the invention may be practiced with the variable equal to any of the values within that range. Thus, for a variable that is inherently discrete, the variable can be equal to any integer value of the numerical range, including the end-points of the range. Similarly, for a variable that is inherently continuous, the variable can be equal to any real value of the numerical range, including the end-points of the range. As an example, a variable which is described as having values between 0 and 2, can be 0; 1 or 2 for variables which are inherently discrete, and can be 0.0, 0.1, 0.01, 0.001, or any other real value for variables which are inherently continuous.

As used herein, unless specifically indicated otherwise, the word "or" is used in the "inclusive" sense of "and/or" and not the "exclusive" sense of "either/or."

Reference is made hereinafter in detail to specific embodiments of the invention. While the invention will be described in conjunction with these specific embodiments, it will be understood that it is not intended to limit the invention to such specific embodiments. On the contrary, it is intended to cover alternatives, modifications, and equivalents as may be included within the spirit and scope of the invention as defined by the appended claims. In the following description, numerous specific details are set forth in order to provide a thorough understanding of the present invention. The present invention may be practiced without some or all of these specific details. In other instances, well known process operations have not been described in detail, in order not to unnecessarily obscure the present invention.

Any suitable materials and/or methods known to those of skill can be utilized in carrying out the present invention. However, preferred materials and methods are described. Materials, reagents and the like to which reference are made in the following description and examples are obtainable from commercial sources, unless otherwise noted.

After experimentation and investigation, it was discovered that when methyl isobutyl ketone was tested as a mobile phase in thin layer chromatography, a satisfactory separation of paclitaxel and cephalomannin was achieved. It was found, however, that the ketone itself was too polar to be used as a mobile phase for the separation of a larger quantity of a paclitaxel mixture, as by column chromatography. The polarity of the mobile phase for column chromatography was then decreased by the addition of one or more less polar solvents. Solvents that are considered less polar and suitable for practicing the present invention include an aliphatic hydrocarbon; an aromatic hydrocarbon; a dialkyl ether; or their mixtures.

The chromatographic process for the separation of paclitaxel and cephalomannin according to the invention is relatively simple. It utilizes normal phase silica gel and does not require high-pressure equipment. Though such equipment can be used as known in the art. With a simple column, however, good separation can be achieved even when only 20 weight parts of silica gel is used for every one part of the starting material. A larger ratio of silica gel can be used depending on the composition of the starting material and desired purity of the resulting isolated paclitaxel. Any silica can be used in practicing the present invention. Silica, or silica gel can be obtained in large quantity from a variety of commercial sources and comes in a variety of particle sizes ranging from about 25 to about 200 µm.

In practicing one aspect of the present invention, a starting paclitaxel composition, *i.e.*, a mixture containing paclitaxel and cephalomannin, is applied to a container, *e.g.*, a column, holding silica. The initial paclitaxel mixture can be obtained from common sources such as from the extract of fresh or dried bark, roots, leaves and/or branches or the whole plant of a paclitaxel containing plant, *e.g.* a *Taxus* plant. Other sources of paclitaxel are also contemplated for use in practicing the present invention, including a crude or purified extract obtained from cell cultures of a cultivated *Taxus* plant, a fermentation broth prepared by cultivation of taxane-producing fungi; a fermentation broth prepared by cultivation of bacterial strains genetically modified for paclitaxel production, etc.

If the starting paclitaxel mixture contains a substantial amount of components in addition to cephalomannin, the mixture can be subjected to purification as is known in the art to reduce the amount of impurities prior to applying the mixture to silica gel chromatography in accordance with the present invention. It is preferred that the starting paclitaxel mixture be a partially purified extract of paclitaxel as obtained by purification steps known in the art, *e.g.*, chromatography, crystallization, etc., but the process described herein is not limited thereto.

When applying the starting paclitaxel composition to the container it can be first diluted with a solvent including the solvent mixture used for its separation. Suitable solvent mixtures that can be used to separate paclitaxel from cephalomannin include methyl isobutyl ketone together with a less polar solvent. In practicing the present invention, the starting paclitaxel mixture, either diluted or concentrated, is applied to the container and the solvent mixture is also applied to the container to cause the paclitaxel and cephalomannin and possibly other components, if present, to partition and separate as the components move through the silica in the container. Highly pure paclitaxel substantially free of cephalomannin can then be collected with the eluting solvent. Additionally, cephalomannin can also be separately collected as well.

In an embodiment of the present invention, the boiling points of the solvents used as the mobile phase should be lower than about 130°C, as the product is preferably recovered from the chromatographic fractions by evaporation. If the boiling points of the solvents are too low however, evaporation of the solvents raises environmental concerns. Hence, the preferred less polar solvents used in practicing embodiments of the present invention include (C₅ - C₈) aliphatic hydrocarbons, such as hexane or heptane; (C₆ - C₈) aromatic hydrocarbons, such as toluene; (C₁- C₄) dialkyl ethers, such as dibutyl ether, diisobutyl ether or *tert*-butyl methyl ether; or a mixture thereof.

Many solvent combinations can be created with methyl isobutyl ketone in practicing the present invention. The majority of these solvent mixtures are commercially available, inexpensive and non-toxic, and thus readily adaptable for industrial-scale use. Any amount of the one or more less polar solvent can be added to methyl isobutyl ketone in practicing the present invention. The ratio of the solvents will depend on several factors including the contents and quantity of the components in the starting paclitaxel composition, the amount of material to be purified, etc. Given the guidance provided herein, those skilled in the art can readily determine the appropriate amount of the one or more less polar solvents to be admixed with the ketone to isolate paclitaxel using no more than routine experimentation. In one embodiment of practicing the present invention, the methyl isobutyl ketone to the less polar solvent comprise the solvent mixture in a volume ratio of about 3:1 (V/V) to about 1:4 (V/V).

A preferred mobile phase for industrial applications is a mixture of methyl isobutyl ketone and toluene. In addition to providing excellent separation of paclitaxel and cephalomannin, this solvent mixture advantageously has a readily recoverable boiling temperature.

After addition of the solvent mixture to a container holding the silica gel and paclitaxel composition, the solvent is eluted from the container and the eluted fractions containing a high concentration of paclitaxel substantially free of cephalomannin are collected. Fractions containing a high concentration of cephalomannin, as well as other components, if present, can also be collected as separate fractions. The paclitaxel fractions, as well as cephalomannin fractions, can then be further isolated by vacuum evaporation of the solvent. The extent of drying will dictate whether the product results in a viscous residue or a solid crystalline mass. If desired, the resulting paclitaxel product can be further purified by crystallization from suitable solvents to obtain paclitaxel in high-purity and in a crystalline form. The use of a completely organic solvent system together with rapid evaporation of the solvents advantageously reduces or eliminates the undesirable isomerization of paclitaxel to 7-*epi*-paclitaxel. This provides another advantage over known isolation methods based on reverse phase chromatography.

### EXAMPLES

The following examples are intended to further illustrate certain preferred embodiments of the invention and are not limiting in nature. Those skilled in the art will recognize, or be able to ascertain, using no more than routine experimentation, numerous equivalents to the specific substances and procedures described herein.

### Example 1

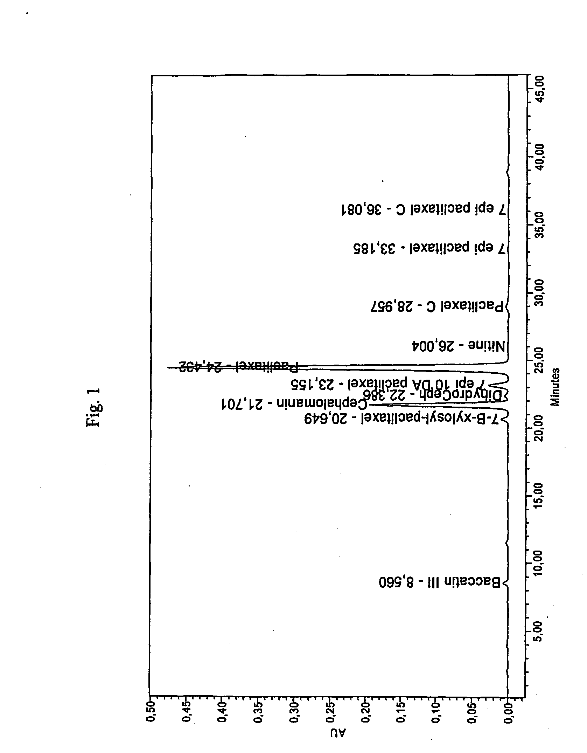

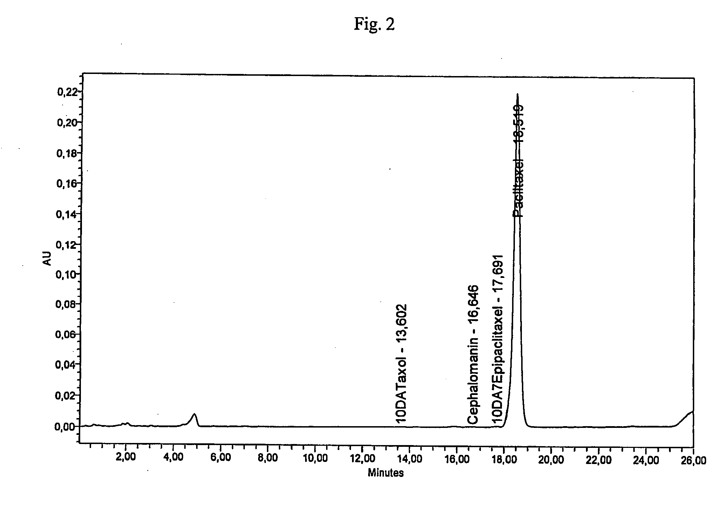

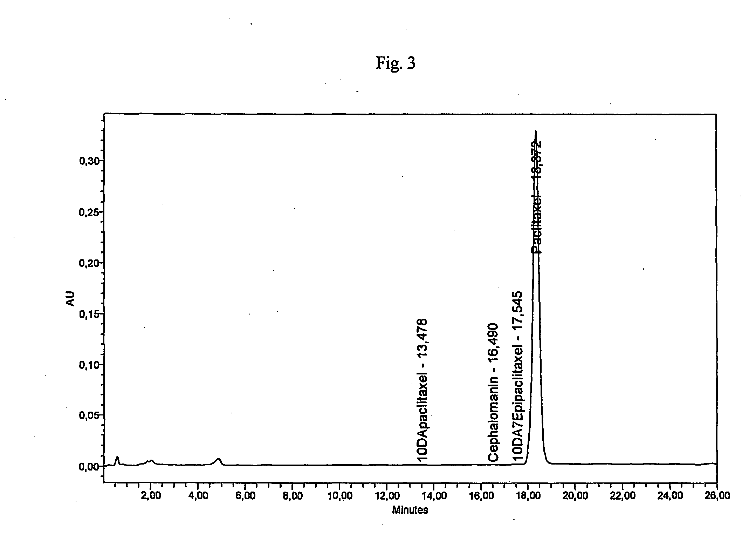

About 99.6 g of crude crystalline paclitaxel composition containing about 55% of paclitaxel and about 19% of cephalomannin (as shown in Fig. 1) was dissolved in about 1000 mL of a methyl isobutyl ketone and toluene (40:60 V/V) mixture. The solution was loaded on a column filled with about 18 kg of silica (silica gel, Merck 60, particle size about 25 - 40 µm). The column was then eluted with a methyl isobutyl ketone and toluene (40:60 V/V) solvent mixture. Fractions of the eluted solvent were taken every 20 L or so and analyzed by HPLC. Fractions containing pure paclitaxel were evaporated to dryness, affording about 59.04 g of dry product containing about 84.035% of paclitaxel and about 0.145% of cephalomannin. The relative ratio between paclitaxel and cephalomannin was 100/0.173 (HPLC analysis shown in Fig. 2). The crystallization of this material from acetone and hexane (1:1 V/V) afforded a crystalline product (45.80 g) containing about 96.272% of paclitaxel and about 0.131 % of cephalomannin (HPLC analysis shown in Fig. 3).

### Example 2

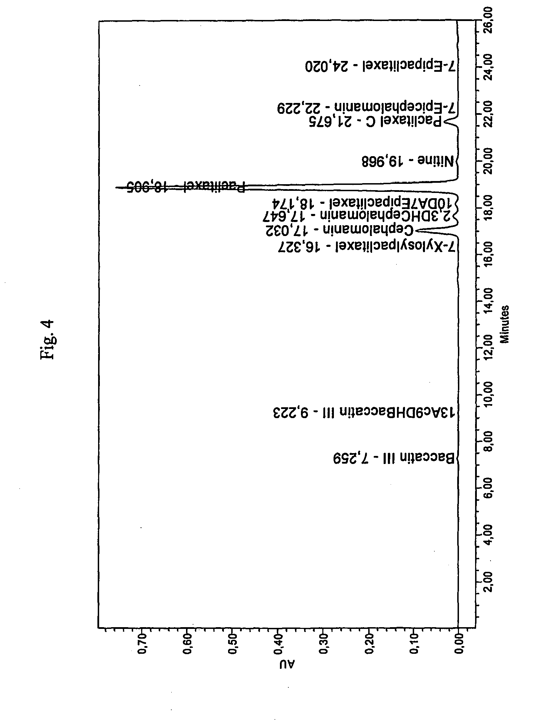

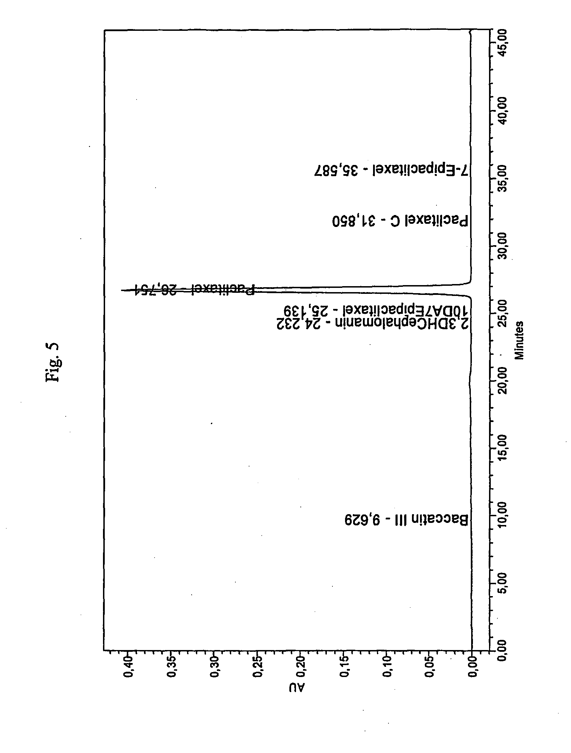

About 1395 g of crude crystalline paclitaxel containing about 72.7% of paclitaxel and about 11.2 % of cephalomannin (as shown in Fig. 4) was dissolved in 20 L of a methyl isobutyl ketone and toluene (40:60 V/V) solvent mixture. The solution was then loaded on a column holding about 185 kg of silica (silica gel, Merck 60, particle size of about 25 - 40 µm). The column was then eluted with a methyl isobutyl ketone and toluene (40:60 V/V) mixture. Fractions of the solution were taken every 100 L and analyzed by HPLC. Fractions containing pure paclitaxel were evaporated to dryness. The dry product contained about 840 g of paclitaxel and about 1.62 g of cephalomannin. The relative ratio between paclitaxel and cephalomannin was 100/0.193. Crystallization of this material from a mixture of acetone and hexane (1:1 V/V) afforded crystalline product (635 g) containing about 96.225% of paclitaxel. The cephalomannin content was lower than the quantification limit of the HPLC detector (HPLC analysis shown in Fig. 5).

### Example 3

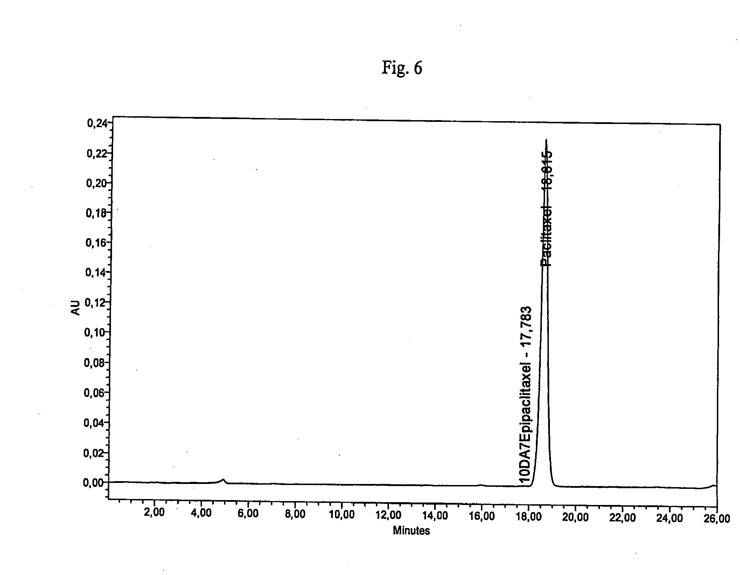

About 4.1 g of crude crystalline paclitaxel containing about 55% of paclitaxel and about 19% of cephalomannin (as shown in Fig. 1) was dissolved in 40 mL of a methyl isobutyl ketone and hexane (70:30 V/V) mixture. The solution was loaded on a column filled with about 150 g of silica (silica gel, Merck 100, particle size of about 63 - 200 µm). The column was then eluted with a methyl isobutyl ketone and hexane 70:30 (V/V) mixture. Fractions of eluted solvent were taken every 200 mL and analyzed by HPLC. Fractions containing pure paclitaxel were evaporated to dryness. The residue (2.4 g) contained about 99.88 % of paclitaxel and about 0.12 % of cephalomannin. The relative ratio between paclitaxel and cephalomannin was 100/0.119 (HPLC analysis in Fig. 6).

### Example 4

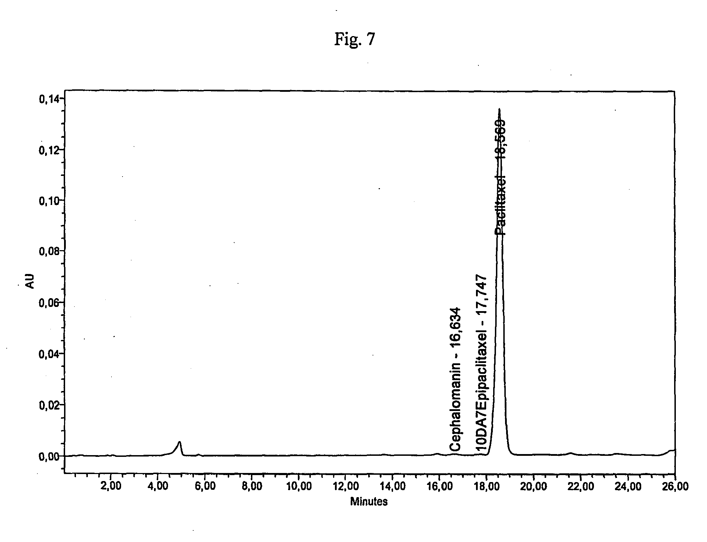

About 4.1 g of crude crystalline paclitaxel containing about 55% of paclitaxel and about 19% of cephalomannin (HPLC analysis in Fig. 3) was dissolved in 40 mL of a methyl isobutyl ketone and *tert*-butyl methyl ether (40:60 V/V) mixture. The solution was loaded on the column filled with 150 g of silica (silica gel, Merck 100, particle size of about 63-200 µm). The column was then eluted with a solvent mixture containing methyl isobutyl ketone and *tert*-butyl methyl ether (40:60 V/V). Fractions of eluted solvent were then taken every 200 mL and analyzed by HPLC. Fractions containing pure paclitaxel were evaporated to dryness. The residue (2.6 g) contained about 99.41 % of paclitaxel and about 0.36 % of cephalomannin. The relative ratio between paclitaxel and cephalomannin was 100/0.458 (HPLC analysis shown in Fig. 7).

In this disclosure there is described only the preferred embodiments of the invention and but a few examples of its versatility. It is to be understood that the invention is capable of use in various other combinations and environments and is capable of changes or modifications within the scope of the inventive concept as expressed herein. Thus, for example, those skilled in the art will recognize, or be able to ascertain, using no more than routine experimentation, numerous equivalents to the specific substances and procedures described herein. Such equivalents are considered to be within the scope of this invention, and are covered by the following claims.

## Claims

1. A process for the separation of paclitaxel and cephalomannin, the process comprising the steps of:
a) applying a paclitaxel and cephalomannin composition to a container containing silica gel;
b) applying to said container a solvent mixture comprising methyl isobutyl ketone mixed with a less polar solvent;
c) eluting the solvent mixture and paclitaxel from the container; and
d) collecting one or more fractions of the eluting solvent mixture containing paclitaxel.

2. The process according to claim 1 wherein the paclitaxel and cephalomannin composition is obtained from the group consisting of an extract of a whole *Taxus* plant, or fresh or dried bark, root, leaf, or branch therefrom

3. The process according to claim 1 wherein the paclitaxel and cephalomannin composition is obtained by extracting cell cultures of a *Taxus* plant.

4. The process according to claim 1 wherein the paclitaxel and cephalomannin composition is obtained by extracting a fermentation broth prepared by cultivation of a taxane-producing fungi.

5. The process according to claim 1 wherein the paclitaxel and cephalomannin composition is obtained by extracting a fermentation broth prepared by cultivation of bacterial strains genetically modified for paclitaxel production.

6. The process according to claim 1 wherein the paclitaxel and cephalomannin composition is a crystalline mixture of paclitaxel and cephalomannin.

7. The process according to claim 1 wherein the less polar solvent is chosen from the group consisting of a C₅ - C₈ aliphatic hydrocarbon, a C₆- C₈ aromatic hydrocarbon, a C₁- C₄ dialkyl ether or a mixture thereof.

8. The process according to claim 7 wherein the C₅ - C₈ aliphatic hydrocarbon is hexane or heptane.

9. The process according to claim 7 wherein the C₆- C₈ aromatic hydrocarbon is toluene.

10. The process according to claim 7 wherein the C₁- C₄ dialkyl ether is dibutyl ether, diisobutyl ether or *tert*-butyl methyl ether.

11. The process according to claim 1 wherein about one part by weight of the paclitaxel and cephalomannin composition is applied to a container containing more than about 20 parts by weight of silica gel.

12. The process according to claim 1 wherein the methyl isobutyl ketone and the less polar solvent comprise the solvent mixture in a volume ratio of about 3:1 (V/V) to about 1:4 (V/V).

13. The process according to claim 12 wherein the less polar solvent is chosen from toluene, hexane, or *tert*-butyl methyl ether.

14. The process according to claim 1 wherein one or more of the collected fractions of the solvent mixture containing paclitaxel contains less than about 0.5% of cephalomannin.

15. The process according to claim 1 further comprising drying one or more eluted fractions of solvent mixture containing paclitaxel to recover crystalline paclitaxel.

16. A process for the separation of paclitaxel and cephalomannin, the process comprising the steps of:
a) applying a paclitaxel and cephalomannin composition to a container containing silica gel;
b) applying to said container a solvent mixture comprising methyl isobutyl ketone mixed with a less polar solvent;
c) eluting the solvent mixture, paclitaxel, and cephalomannin from the container;
d) collecting one or more fractions of the eluting solvent mixture containing a high concentration of paclitaxel; and
e) collecting one or more fractions of the eluting solvent mixture containing a high concentration of cephalomannin.

## Patentansprüche

1. Verfahren zum Trennen von Paclitaxel und Cephalomannin, wobei das Verfahren die folgenden Stufen umfaßt:
a) Einbringen einer Zusammensetzung aus Paclitaxel und Cephalomannin in einen Behälter, der Silicagel enthält,
b) Einbringen eines Lösungsmittelgemischs, welches Methylisobutylketon im Gemisch mit einem weniger polaren Lösungsmittel umfaßt, in den Behälter,
c) Eluieren des Lösungsmittelgemischs und von Paclitaxel aus dem Behälter und
d) Sammeln einer oder mehrerer Fraktionen des Elutionslösungsmittelgemischs, die Paclitaxel enthält bzw. enthalten.

2. Verfahren nach Anspruch 1, wobei die Zusammensetzung aus Paclitaxel und Cephalomannin aus der Gruppe erhalten wird, bestehend aus einem Extrakt von einer ganzen *Taxus-*Pflanze oder von frischer oder getrockneter Rinde, einer Wurzel, einem Blatt oder einem Zweig davon.

3. Verfahren nach Anspruch 1, wobei die Zusammensetzung aus Paclitaxel und Cephalomannin durch Extrahieren von Zellkulturen einer Taxus-Pflanze erhalten wird.

4. Verfahren nach Anspruch 1, wobei die Zusammensetzung aus Paclitaxel und Cephalomannin durch Extrahieren einer Fermentationsbrühe, hergestellt durch Kultivieren eines Taxanerzeugenden Pilzes, erhalten wird.

5. Verfahren nach Anspruch 1, wobei die Zusammensetzung aus Paclitaxel und Cephalomannin durch Extrahieren einer Fermentationsbrühe, erhalten durch Kultivieren von Bakterienstämmen, die für die Produktion von Paclitaxel genetisch modifiziert sind, erhalten wird.

6. Verfahren nach Anspruch 1, wobei die Zusammensetzung aus Paclitaxel und Cephalomannin ein kristallines Gemisch von Paclitaxel und Gephalomannin ist.

7. Verfahren nach Anspruch 1, wobei das weniger polare Lösungsmittel aus der Gruppe ausgewählt ist, bestehend aus einem aliphatischen C₅-C₈-Kohlenwasserstoff, einem aromatischen C₆-C₈-Kohlenwasserstoff, einem C₁-C₄-Dialkylether oder einem Gemisch davon.

8. Verfahren nach Anspruch 7, wobei der aliphatische C₅-C₈-Kohlenwasserstoff Hexan oder Heptan ist.

9. Verfahren nach Anspruch 7, wobei der aromatische C₆-C₈-Kohlenwasserstoff Toluen ist.

10. Verfahren nach Anspruch 7, wobei der C₁-C₄-Dialkylether Dibutylether, Diisobutylether oder *tert*-Butylmethylether ist.

11. Verfahren nach Anspruch 1, wobei etwa ein Gewichtsteil der Zusammensetzung von Paclitaxel und Cephalomannin in einen Behälter eingebracht wird, der mehr als etwa 20 Gewichtsteile Silicagel enthält.

12. Verfahren nach Anspruch 1, wobei das Methylisobutylketon und das weniger polare Lösungsmittel das Lösungsmittelgemisch in einem Volumenverhältnis von etwa 3:1 (VN) bis etwa 1:4 (VN) bilden.

13. Verfahren nach Anspruch 12, wobei das weniger polare Lösungsmittel unter Toluen, Hexan oder *tert*-Butylmethylether ausgewählt ist.

14. Verfahren nach Anspruch 1, wobei eine oder mehrere der gesammelten Fraktionen des Lösungsmittelgemischs, die Paclitaxel enthält bzw. enthalten, weniger als etwa 0,5% an Cephalomannin enthält bzw. enthalten.

15. Verfahren nach Anspruch 1, welches weiterhin das Trocknen einer oder mehrerer eluierter Fraktionen von Lösungsmittelgemisch, die Paclitaxel enthält bzw. enthalten, umfaßt, um kristallines Paclitaxel zu gewinnen.

16. Verfahren zum Trennen von Paclitaxel und Cephalomannin, wobei das Verfahren die folgenden Stufen umfaßt:
a) Einbringen einer Zusammensetzung von Paclitaxel und Cephalomannin in einen Behälter, der Silicagel enthält,
b) Einbringen eines Lösungsmittelgemischs, welches Methylisobutylketon im Gemisch mit einem weniger polaren Lösungsmittel umfaßt, in den Behälter,
c) Eluieren des Lösungsmittelgemischs, von Paclitaxel und Cephalomannin aus dem Behälter,
d) Sammeln einer oder mehrerer Fraktionen des Elutionslösungsmittelgemischs, die eine hohe Konzentration von Paclitaxel enthält bzw. enthalten, und
e) Sammeln einer oder mehrerer Fraktionen des Elutionslösungsmittelgemischs, die eine hohe Konzentration von Cephalomannin enthält bzw. enthalten.

## Revendications

1. Un procédé pour la séparation de paclitaxel et de céphalomannine, ce procédé comprenant les étapes consistant à: _
a) appliquer une composition de paclitaxel et de céphalomannine à une capsule contenant du silica gel
b) appliquer à ladite capsule un mélange de solvant comprenant de la méthyl isobutyl cétone mélangée à un solvant moins polaire,
c) éluer le mélange de solvant et de paclitaxel de la capsule; et
d) recueillir une ou plusieurs fractions du mélange de solvant d'élution contenant le paclitaxel

2. Le procédé selon la revendication 1, dans lequel la composition de paclitaxel et de céphalomannine est produite à partir de produits choisis dans le groupe comprenant les extraits d'ifs (*Taxus*) ou d'écorces, de racines, de feuilles ou de branches séchées ou fraîches d'ifs

3. Le procédé selon la revendication 1, dans lequel la composition de paclitaxel et de céphalomannine est obtenue par extraction de cultures de cellules d'ifs (*taxus*)

4. Le procédé selon la revendication 1, dans lequel la composition de paclitaxel et de céphalomannine est obtenue par extraction d'un bouillon de fermentation préparé par culture d'un champignon producteur de taxane.

5. Le procédé selon la revendication 1, dans lequel la composition de paclitaxel et de céphalomannine est obtenue par extraction d'un bouillon de fermentation préparé par culture de souches bactériennes génétiquement modifiées convenant pour la production de paclitaxel.

6. Le procédé selon la revendication 1, dans lequel la composition de paclitaxel et de céphalomannine est un mélange cristallisé de paclitaxel et de céphalomannine.

7. Le procédé selon la revendication 1, dans lequel le solvant moins polaire est choisi dans le groupe comprenant les hydrocarbures aliphatiques en C₅-C₆, les hydrocarbures aromatiques en C₆-C₈, les (dialkyl en C₁-C₄)-éther ou un de leurs mélanges.

8. Le procédé selon la revendication 7, dans lequel les hydrocarbures aliphatiques en C₅-C₈ consistent en hexane ou heptane.

9. Le procédé selon la revendication 7, dans lequel les hydrocarbures aromatiques en C₆-C₈ consistent en toluène.

10. Le procédé selon la revendication 7, dans lequel les (dialkyl en C₁-C₄)-éther consistent en diisobutyl éther ou tert.-butyl méthyl éther.

11. Le procédé selon la revendication 1, dans lequel environ 1 partie en poids de la composition de paclitaxel et de céphalomannine est appliquée à une capsule contenant plus d'environ 20 parties en poids de silica gel.

12. Le procédé selon la revendication 1, dans lequel la méthyl isobutyl cétone et solvant moins polaire comprend le mélange de solvant dans un rapport volumique d'environ 3/1 (v/v) à environ 1/4 (v/v).

13. Le procédé selon la revendication 12, dans lequel le solvant moins polaire consiste en toluène, hexane ou tert-butyl méthyl éther.

14. Le procédé selon la revendication 1, dans lequel une ou plusieurs fractions de mélange de solvant contenant du paclitaxel recueillies contient moins d'environ 0,5% de céphalomannine.

15. Le procédé selon la revendication 1, comprenant en outre le séchage de une ou plusieurs fractions de mélange de solvant contenant du paclitaxel éluées pour récupérer de paclitaxel crystallisé.

16. Un procédé pour la séparation de paclitaxel et de céphalomannine, ce procédé comprenant les étapes consistant à:
a) appliquer une composition de paclitaxel et de céphalomannine_à une capsule contenant du silica gel
b) appliquer à ladite capsule un mélange de solvant comprenant de la méthyl isobutyl cétone mélangée à un solvant moins polaire,
c) éluer le mélange de solvant, de paclitaxel et de céphalomannine de la capsule;
d) recueillir une ou plusieurs fractions du mélange de solvant d'élution contenant une forte concentration de paclitaxel
e) recueillir une ou plusieurs fractions du mélange de solvant d'élution contenant une forte concentration de céphalomannine.
